# EUROPEAN PATENT APPLICATION

(11) **EP 4 098 289 A1**
(43) Date of publication of application: **07.12.2022**
(21) Application number: 21784842.3
(22) Date of filing: 23.02.2021
(51) Int. Cl.: A61M 1/00, A61M 25/14

(54) **DISPOSABLE FLUSHING AND SUCTION DRAINAGE TUBE AND USING METHOD THEREOF**

(30) Priority: 10.04.2020 CN 202010280226
(71) Applicant: Beijing Honghugaoxiang Technology Development Co., Ltd, Beijing 102600 (CN)
(72) Inventor: GUO, Dongliang, Beijing 102600 (CN); WANG, Bing, Beijing 102600 (CN)
(74) Representative: Chung, Hoi Kan
(86) International application number: PCT/CN2021/077395
(87) International publication number: WO 2021/203855

(57) **Abstract**

A disposable flushing and suction drainage tube and a using method thereof, relating to the technical field of medical instruments. The flushing and suction drainage tube comprises a flushing tube (1), a suction tube (2), and a mounting base (3). The flushing tube (1) and the suction tube (2) are respectively mounted at one end of the mounting base (3), and an air connector (4) and a water connector (5) are mounted at the other end; the mounting base (3) comprises a tube body base (31) and a control base (32), the flushing tube (1) and the suction tube (2) are mounted at one end of the tube body base (31), and the other end is detachably connected to the control base (32). The disposable flushing and suction drainage tube can achieve control of suction for the suction tube (2) by means of a negative pressure adjusting opening (6) on the mounting base (3); opening, closing, and water flow of the flushing tube (1) is controllable by means of a water control switch (324) on the mounting base (3), the position of the negative pressure adjusting opening (6) is close to the position of the water control switch (324), one-hand operation is facilitated, switching between flushing and drainage and the flow of drainage can be controlled, and the operation is simple.

## Description

The present application claims priority from the Chinese Patent Application No. 202010280226.7 filed to the Chinese Patent Office on April 10, 2020 and titled "A Kind of Disposable Flushing and Suction Drainage Tube and Its Operation Method", the entire content of which is incorporated herein by reference.

### Field of the Invention

The present application relates to the field of the invention of medical devices, and more particularly, to a disposable flushing and suction drainage tube and its operation method.

### Background of the Invention

During a surgical operation, it is necessary to wash the operative region or instruments with stroke-physiological saline solution and to timely conduct the suction drainage of bloody liquid from the wound or flushing water, thus clearing the filth in the operative field, making the operative field clear, and reducing the infection possibility or infected area. During a surgical operation, the flushing and drainage, which are usually assisted by assistants, require the control of conversion between flushing and drainage, as well as the control of drainage flow.

At present, the existing flushing and drainage devices require the simultaneous cumbersome operation of both hands. Sometimes, due to the limitations of site and personnel, the doctor needs a simplified operation, for example, an operation by himself/herself. However, the structures of existing flushing and drainage devices make it impossible to use only one hand to control the conversion between flushing and drainage, as well as the drainage flow.

### Scope of the Application

For this purpose, the present application provides a kind of disposable flushing and suction drainage tube and its operation method to solve the problems of existing flushing and drainage devices such as cumbersome operation and impossibility of single-hand control.

To achieve the above-mentioned purpose, the embodiments of the present application provide the following technical schemes:
As the first aspect, the embodiments of the present application provide a kind of disposable flushing and suction drainage tube, which is characterized in that it comprises a flushing tube, a suction tube, and a mounting base; one end of the mounting base is connected with the flushing tube and the suction tube, while the other end is connected with a gas joint and a water joint; the mounting base comprises a tube body base and a control base; one end of the tube body base is installed with the flushing tube and the suction tube, while the other end is detachably connected with the control base.

The control base comprises a control base body, a water-flowing inner tube, a water locking pin roll, and a water control switch. The water control switch is slidably mounted onto the control base body, the water locking pin roll is movably mounted inside the control base body, the water-flowing inner tube is mounted inside the control base body, the water locking pin roll and the water control switch cooperate with each other during use to control the water flow in the water-flowing inner tube; and the control base body is equipped with a negative pressure control port.

Further, a water-flowing inner cavity and a first gas-flowing inner cavity are arranged inside the tube body base, the end of the tube body base distal from the flushing tube is connected with a water route connecting base and a gas route connecting base.

Further, a second gas-flowing inner cavity connected with the first gas-flowing inner cavity is arranged inside the gas route connecting base; a connecting guide rail and an annular limiting bulge are arranged outside the gas route connecting base; and the gas route connecting base is arranged with a notch corresponding to the position of the negative pressure control port.

Further, a negative pressure channel connected with the second gas-flowing inner cavity is arranged in the control base body, a positioning groove corresponding to the connecting guide rail and a groove corresponding to the limiting bulge are arranged inside one end of the negative pressure channel, while the gas joint is connected to the other end.

Further, an inner tube groove is arranged inside the control base body, the water-flowing inner tube is installed in the inner tube groove; one end of the water-flowing inner tube is installed on the water route connecting base, while the other end is connected to the water joint; both sides of the inner tube groove are arranged with the longitudinally opened first guide slot and second guide slot, one end of the water locking pin roll is installed inside the first guide slot, while the other end is installed inside the second guide slot.

Further, the flushing and suction drainage tube further comprises a lighting device; the lighting device comprises a light emitting diode, a battery, and an optical fiber cable; the light emitting diode, battery, and optical fiber cable are connected by electric circuits; the light emitting diode and the battery are installed in the tube body base, the front end of the tube body base is arranged with a light hole, one end of the optical fiber cable passes through the light hole and then is connected to the light emitting diode, while the other end extends in the direction away from the tube body base.

The positive and negative pins of the light emitting diode are connected to the positive and negative poles of the battery correspondingly; one pin of the light emitting diode is installed with an elastic pin; the elastic pin and the battery are separated by an insulating strip; during the use, the insulating strip is removed to make the elastic pin contact the positive and negative poles of the battery, thus enabling the battery to energise the light emitting diode to emit light.

The position on the tube body base corresponding to the battery is installed with a stop block.

Further, the control base further comprises a fixing shell; the fixing shell is detachably mounted onto the control base body, the fixing shell and the control base body form a mounting groove, and the water control switch is installed in the mounting groove.

Further, the water control switch comprises an operating panel, a mounting slider, and a pin roll control block; the operating panel, mounting slider, and pin roll control block are connected successively from up to down; the pin roll control block is an inverted rectangular trapezoidal block, which comprises an oblique isosceles surface and a lower bottom surface, and the bottom surface of the pin roll control block has an opening.

Further, the front end of the flushing tube is sloped and seperated 5-35 mm from the front end of the suction tube; the flushing tube is also connected to a casing pipe, and the casing pipe is arranged outside the suction tube.

As the second aspect, the embodiments of the present application provide the method of operating the above-mentioned flushing and suction drainage tube; the operation method comprises the following steps:
connect the gas j oint to the negative pressure source, and connect the water j oint to the water source.
when flushing is required, slide the water control switch to the "ON" end; the water control switch slides to change from the state where its lower bottom surface contacts the water locking pin roll to the state where its oblique isosceles surface contacts the water locking pin roll, the water locking pin roll moves upward under the elastic force of the water-flowing inner tube, the water-flowing inner tube changes from the "Closed" state to the "OPEN" state, the water flows through the water joint and the water-flowing inner tube and reaches the flushing tube to start the flushing; adjust the position of the water control switch, adjust the position where the oblique isosceles surface of the water control switch contacts the water locking pin roll, control the upward movement distance of the water locking pin roll, and control the flattening degree of the water-flowing inner tube, thus realizing the water flow control. When flushing needs to be stopped, slide the water control switch to the "OFF" end; the water control switch slides to change from the state where its oblique isosceles surface contacts the water locking pin roll to the state where its lower bottom surface contacts the water locking pin roll; the water locking pin roll moves downward to flatten the water-flowing inner tube, and the water-flowing inner tube changes from the "OPEN" state to the "Closed" state, thus stopping the water flow.
when suction is required, use a finger to block the negative pressure control port to drain the waste fluid and flushing fluid, and control the suction force of the suction tube by the size of the blocked negative pressure control port.

The present application has the following advantages:
The disposable flushing and suction drainage tube under the present application is a disposable medical supply that comprises a flushing tube, a suction tube, and a mounting base. The negative pressure control port on the mounting base can realize control of the suction force of the suction tube, and the water control switch on the mounting base can realize control of the "ON" and "OFF" of the flushing tube and the water flow. In addition, the negative pressure control port is near the water control switch, which facilitates the single-hand operation to control the conversion between flushing and drainage, as well as the drainage flow, thus making the operation simple.

The disposable flushing and suction drainage tube under the present application is further arranged with a lighting device for lighting the operative site, thus making the operative field clearer.

### Brief Description of the Drawings

To clarify the embodiments of the present application or the technical schemes in the prior art more clearly, the attached drawings necessary in the description of the embodiments or the prior art are briefly introduced as follows: Apparently, the drawings in the following description are exemplary, and those of ordinary skill in the art can derive and obtain other drawings from these drawings provided herein without creative efforts.

Structures, proportions, and sizes indicated in the specifications are the content disclosed for matching the specifications, so as to understand and read by persons skilled in the art, rather than defining the restrictive conditions that the present application can implement, therefore, they have no technically substantive meaning. Modification of any structure, change in proportional relation, or size adjustment should fall in the scope covered by the technical content disclosed by the present application without affecting the effect generated by the present application and the purpose achieved by the application.
FIG. 1 is an overall structural view of a kind of disposable flushing and suction drainage tube provided in Embodiment 1 of the present application;
FIG. 2 is an exploded view of a kind of disposable flushing and suction drainage tube provided in Embodiment 1 of the present application;
FIG. 3 is an internal structural view of a kind of disposable flushing and suction drainage tube provided in Embodiment 1 of the present application;
FIG. 4 is a three-dimensional structural view of a tube body base provided in Embodiment 1 of the present application;
FIG. 5 is a three-dimensional structural view of a control base body provided in Embodiment 1 of the present application;
FIG. 6 is a structural view of a water-flowing inner tube provided in Embodiment 1 of the present application;
FIG. 7 is a view for the cooperative use of the water locking pin roll and the water control switch provided in Embodiment 1 of the present application during flushing;
FIG. 8 is a view for the cooperative use of the water locking pin roll and the water control switch provided in Embodiment 1 of the present application upon the termination of flushing;
FIG. 9 is a structural schematic view of the lighting device on the flushing and suction drainage tube provided in Embodiment 2 of the present application;
FIG. 10 is an installation schematic view of the installation of optical fiber cable onto the tube body base provided in Embodiment 2 of the present application;
FIG. 11 is a structural schematic view of the tube body base of the flushing and suction drainage tube provided in Embodiment 2 of the present application;

In the drawings: flushing tube 1, suction tube 2, mounting base 3, tube body base 31, water-flowing inner cavity 311, first gas-flowing inner cavity 312, control base 32, control base body 321, water-flowing inner tube 322, tube body 3221, mounting joint 3222, water locking pin roll 323, water control switch 324, operating panel 3241, mounting slider 3242, pin roll control block 3243, fixing shell 325, gas joint 4, water joint 5, negative pressure control port 6, water route connecting base 7, gas route connecting base 8, second gas-flowing inner cavity 80, notch 81, connecting guide rail 9, limiting bulge 10, negative pressure channel 11, positioning groove 110, inner tube groove 12, first guide slot 13, second guide slot 14, oblique isosceles surface 15, lower bottom surface 16, opening 17, casing pipe 18, mounting positioning hole 19, mounting plug-in 20, lighting device 21, light emitting diode 211, battery 212, optical fiber cable 213, tube body base 31, light hole 22, elastic pin 23, insulating strip 24, stop block 25.

### Detailed Description of the Preferred Embodiments

The following specific embodiments give a detailed description of the preferred embodiments in the present application. The persons skilled in the art can easily understand other advantages and effects of the present application based on the content in the Specifications. Apparently, the embodiments are only some of rather than all of the embodiments of the present application. Based on the embodiments described in the present application, all other embodiments obtained by those of ordinary skill in the art without creative efforts shall fall within the scope of the present application.

### Embodiment 1

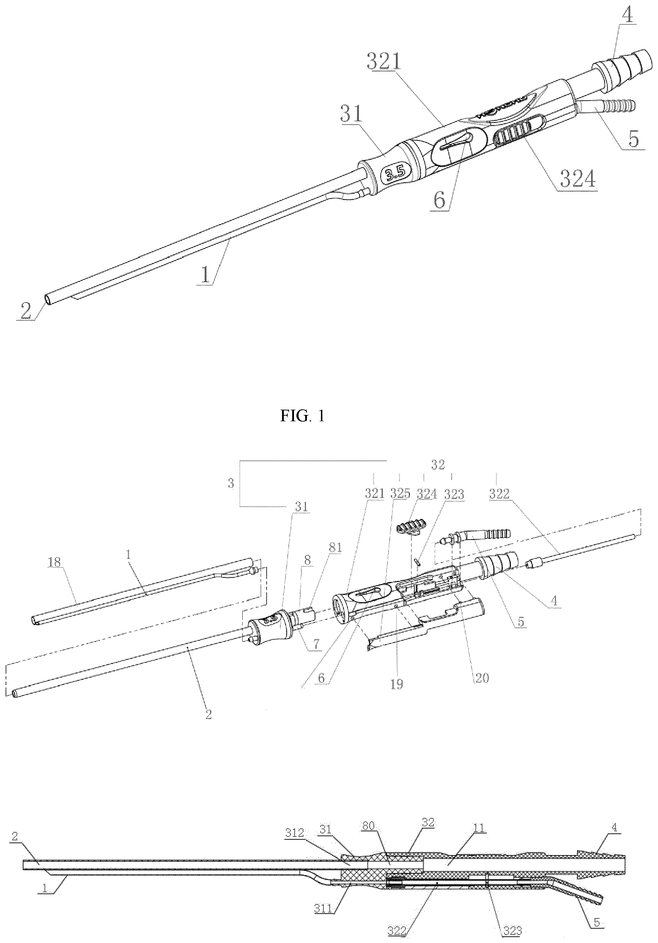

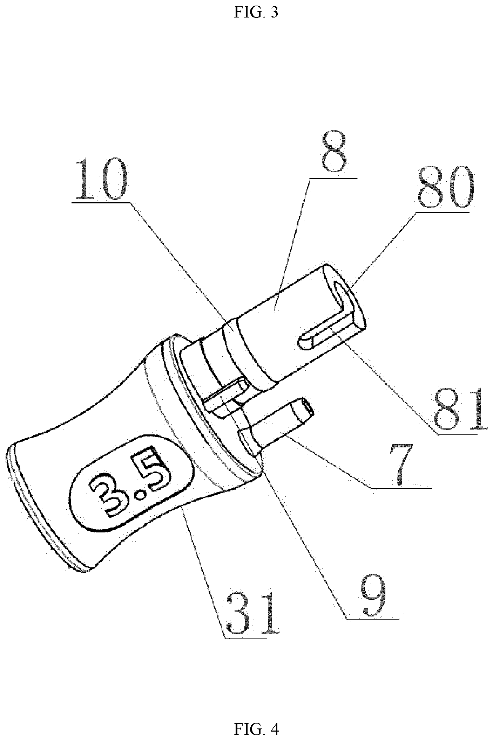

As illustrated in FIG. 1- FIG. 5, this embodiment describes a kind of disposable flushing and suction drainage tube, which is characterized in that it comprises a flushing tube 1, a suction tube 2, and a mounting base 3; one end of the mounting base 3 is connected with the flushing tube 1 and the suction tube 2, while the other end is connected with a gas joint 4 and a water joint 5; the suction tube 2 is made of soft stainless steel tube, which can be bent by a doctor according to the operation needs to any angle and state, and the front end of which was rounded to avoid any harm to patients; the flushing tube 1 is made of silica gel, PVC, or stainless steel and arranged side by side with suction tube 2; the front end of the flushing tube 1 is sloped and separated 5-35 mm from the front end of the suction tube 2; the flushing tube 1 is also connected to a casing pipe 18, and the casing pipe 18 is arranged outside the suction tube 2.

The mounting base 3 comprises a tube body base 31 and a control base 32; one end of the tube body base 31 is installed with the flushing tube 1 and the suction tube 2, while the other end is detachably connected with the control base 32; the tube body base 31 has several specifications for the suction tube 2 of different dimensions and specifications according to the operation needs; the control base 32 is the part held by the operator and it is a functional component.

The control base 32 comprises a control base body 321, a water-flowing inner tube 322, a water locking pin roll 323, a water control switch 324, and a fixing shell 325. The fixing shell 325 is detachably mounted onto the control base body 321, and the fixing shell 325 is arranged with multiple mounting plug-ins 20. The control base body 321 is arranged with multiple mounting positioning holes 19 matching the mounting plug-ins 20. The fixing shell 325 and the control base body 321 form a mounting groove, and the water control switch 324 is installed in the mounting groove and can slide front and back along the mounting groove. The water locking pin roll 323 is movably mounted inside the control base body 321, the water-flowing inner tube 322 is mounted inside the control base body 321, the water locking pin roll 323 and the water control switch 324 cooperate with each other during use to control the water flow in the water-flowing inner tube 322. The upward and downward movement of the water locking pin roll 323 can squeeze or release the water-flowing inner tube 322, thus opening or closing the water-flowing inner tube 322 and controlling the water flow.

The control base body 321 is equipped with a negative pressure control port 6. The negative pressure control port 6 can control the suction force of the suction tube 2 to realize the suction. Further, the negative pressure control port 6 is designed as an arc that matches the thumb shape to facilitate the operation. The negative pressure control port 6 is near the water control switch 324, which facilitates the single-hand operation.

A water-flowing inner cavity 311 and a first gas-flowing inner cavity 312 are arranged inside the tube body base 31, the end of the tube body base 31 away from the flushing tube 1 is connected with a water route connecting base 7 and a gas route connecting base 8. The water route connecting base 7 and the gas route connecting base 8 can realize the plug-in connection between the tube body base 31 and the control base 32 and ensure the smoothness of both water route and gas route.

A second gas-flowing inner cavity 80 connected with the first gas-flowing inner cavity 312 is arranged inside the gas route connecting base 8; a connecting guide rail 9 and an annular limiting bulge 10 are arranged outside the gas route connecting base 8; and the gas route connecting base 8 is arranged with a notch 81 corresponding to the position of the negative pressure control port 6. A negative pressure channel 11 connected with the second gas-flowing inner cavity 80 is arranged in the control base body 321, a positioning groove 110 corresponding to the connecting guide rail 9 and a groove corresponding to the limiting bulge 10 are arranged inside one end of the negative pressure channel 11, while the gas joint 4 is connected to the other end. The connecting guide rail 9, the positioning groove 110, the limiting bulge 10, and the groove perfectly realize the plug-in connection between the tube body base 31 and the control base 32, and prevent the relative rotation between the tube body base 31 and the control base 32.

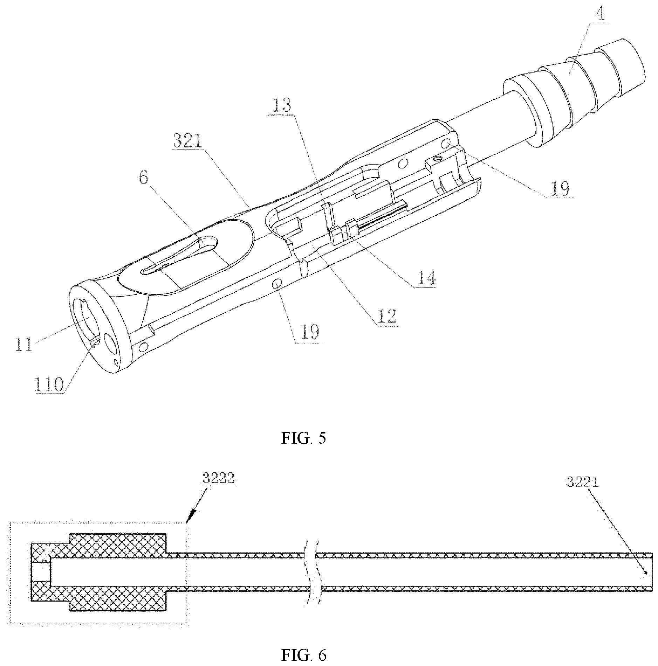

An inner tube groove 12 is arranged inside the control base body 321, the water-flowing inner tube 322 is installed in the inner tube groove 12; one end of the water-flowing inner tube 322 is installed on the water route connecting base 7, while the other end is connected to the water joint 5. As illustrated in FIG. 6, the water-flowing inner tube 322 comprises a tube body 3221 and a mounting joint 3222. The mounting joint 3222 is clamped into the positioning hole of the inner tube groove 12. The aperture of the end of inner cavity of the mounting joint 3222 away from the tube body matches the outer diameter of the water route connecting base 7 to prevent water leakage.

Both sides of the inner tube groove 12 are arranged with the longitudinally opened first guide slot 13 and second guide slot 14, one end of the water locking pin roll 323 is installed inside the first guide slot 13, while the other end is installed inside the second guide slot 14. The first guide slot 13 and the second guide slot 14 in this embodiment are structures of the same inner diameter and can control the upward and downward movement of the water locking pin roll 323. The first guide slot 13 and the second guide slot 14 in the present application are not limited to the shapes mentioned in this embodiment. Instead, they can be designed as chutes with gradually increasing inner diameter from bottom to top.

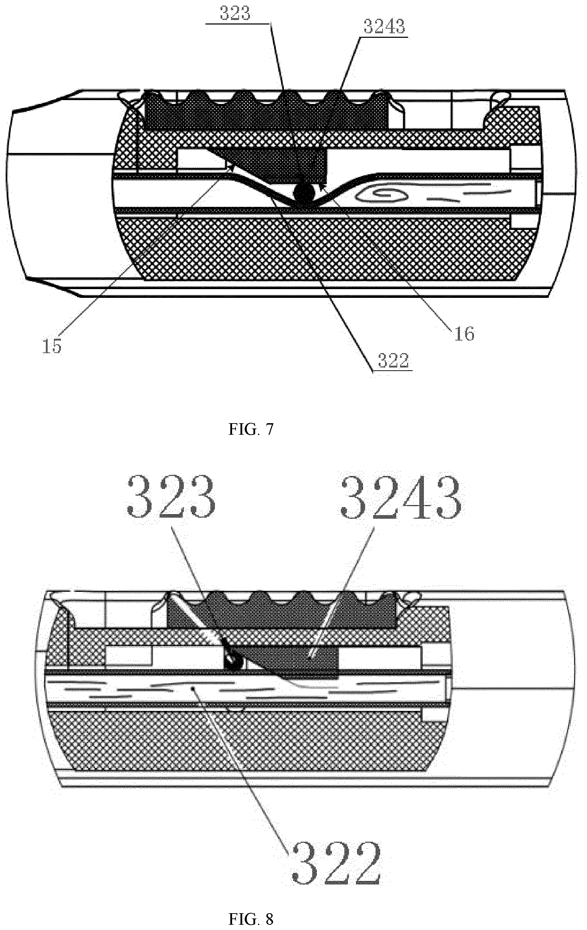

The water control switch 324 comprises an operating panel 3241, a mounting slider 3242, and a pin roll control block 3243; the operating panel 3241, mounting slider 3242, and pin roll control block 3243 are connected successively from up to down; the mounting slider 3242 is installed inside the mounting groove formed by the fixing shell 325 and the control base body 321, and can slide back and forth inside the mounting groove; the pin roll control block 3243 is an inverted rectangular trapezoidal block, which comprises an oblique isosceles surface 15 and a lower bottom surface 16, and the bottom surface of the pin roll control block 3243 has an opening 17. The operating panel 3241 is designed in a wave shape, which can increase the friction between the hand and the operating panel 3241, prevent slippage during operation, and reduce mistakes. The opening 17 makes way for the water-flowing inner tube 322 and allows the water-flowing inner tube 322 to pass through. The oblique isosceles surface 15 is a functional guide slope to control the distance of upward/downward movement of the water locking pin roll 323, thus controlling the water flow. The principle of how the water control switch 324 and the water locking pin roll 323 cooperate with each other to control the water flow is as follows: the water control switch 324 can be pushed to the "ON" end and "OFF" end, as illustrated in FIG. 7 and FIG. 8; during flushing, the oblique isosceles surface 15 of the pin roll control block 3243 presses against the water locking pin roll 323 to make it move up and down to control the water flow inside the water-flowing inner tube 322; upon the termination of flushing, the lower bottom surface 16 of the pin roll control block 3243 presses against the water locking pin roll 323 to make the water locking pin roll 323 to the very bottom, thus flattening and closing the water-flowing inner tube 322 and stopping the water flow; when the water control switch 324 moves between the "ON" end and "OFF" end, the water flow can be adjusted to the required value.

### Embodiment 2

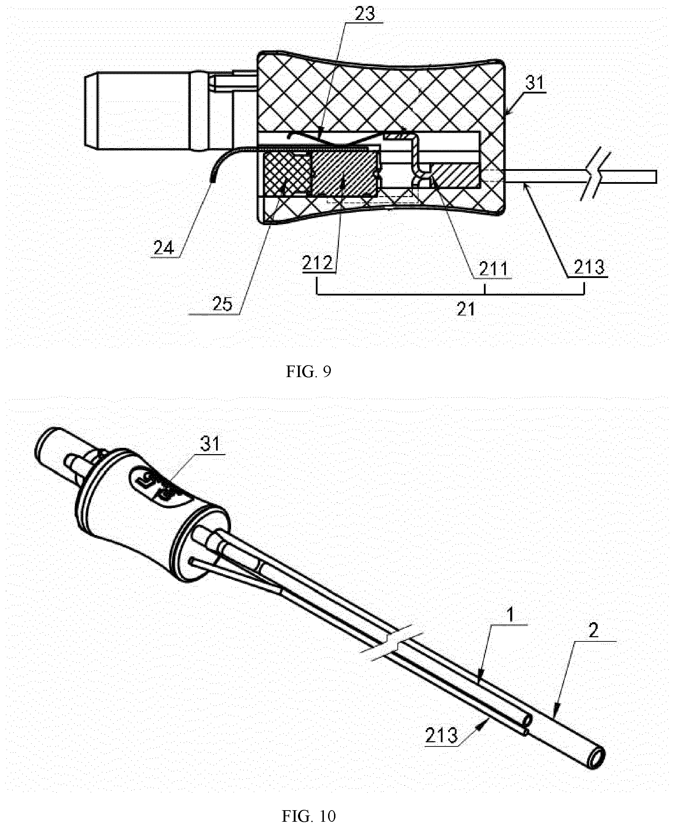

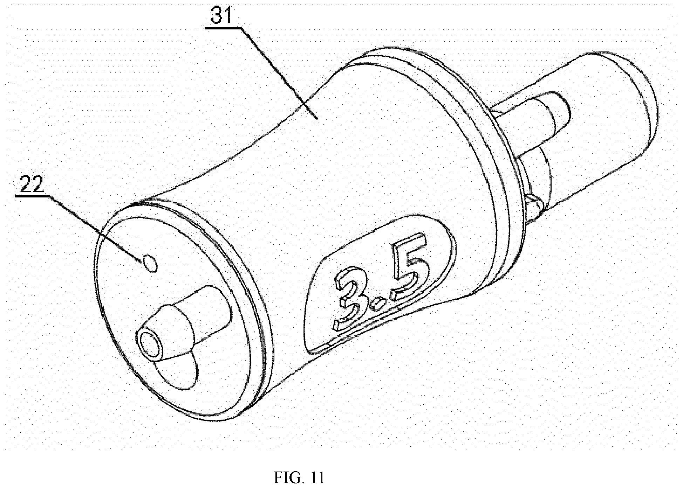

This embodiment describes a kind of disposable flushing and suction drainage tube, which is characterized in that it not only has the technical characteristics described in Embodiment 1 but also has the following technical characteristics:
As shown in FIG. 9 to FIG. 11, the flushing and suction drainage tube further comprises a lighting device 21 for lighting the operative site, thus making the operative field clearer. The lighting device 21 comprises a light emitting diode 211, a battery 212, and an optical fiber cable 213; the light emitting diode 211, battery 212, and optical fiber cable 213 are connected by electric circuits; the light emitting diode 211 and the battery 212 are installed in the tube body base 31, the front end of the tube body base 31 is arranged with a light hole 22, one end of the optical fiber cable 213 passes through the light hole 22 and then is connected to the light emitting diode 211, while the other end extends in the direction away from the tube body base 31; when the battery 212 is turned on, the light emitting diode 211 emits the light, the light propagates through the optical fiber cable 213 to the head end of the flushing and suction drainage tube for lighting the operative site.

The positive and negative pins of the light emitting diode 211 are connected to the positive and negative poles of the battery 212 correspondingly; one pin of the light emitting diode 211 is installed with an elastic pin 23; the elastic pin 23 and the battery 212 are separated by an insulating strip 24; during the use, the insulating strip 24 is removed to make the elastic pin 23 contact the positive and negative poles of the battery 212, thus enabling the battery 212 to energise the light emitting diode 211 to emit light.

The position on the tube body base 31 corresponding to the battery 212 is installed with a stop block 25. The stop block 25 can stably fix the battery 212 inside the tube body base 31.

### Embodiment 3

The operation method of the flushing and suction drainage tube described in Embodiment 1 includes the following steps:
Connect the gas joint 4 to the negative pressure source, and connect the water joint 5 to the water source.

When flushing is not performed, the water control switch 324 in at the "OFF" end, the lower bottom surface 16 of the pin roll control block 3243 presses against the water locking pin roll 323 to locate the water locking pin roll 323 at the very bottom of the first guide slot 13 and the second guide slot 14; when flushing is required, push the operating panel 3241 to slide the water control switch 324 to the "ON" end; the pin roll control block 3243 of the water control switch 324 slides to change from the state where its lower bottom surface 16 contacts the water locking pin roll 323 to the state where its oblique isosceles surface 15 contacts the water locking pin roll 323, the water locking pin roll 323 moves upward under the elastic force of the water-flowing inner tube 322, the water-flowing inner tube 322 changes from the "Closed" state to the "OPEN" state, the water flows through the water joint 5 and the water-flowing inner tube 322 and reaches the flushing tube 1 to start the flushing; adjust the position of the water control switch 324, adjust the position where the oblique isosceles surface 15 of the pin roll control block 3243 contacts the water locking pin roll 323, control the upward movement distance of the water locking pin roll 323, and control the flattening degree of the water-flowing inner tube 322, thus realizing the water flow control. When flushing needs to be stopped, operate the operating panel 3241 to slide the water control switch 324 to the "OFF" end; the pin roll control block 3243 of the water control switch 324 slides to change from the state where its oblique isosceles surface 15 contacts the water locking pin roll 323 to the state where its lower bottom surface 16 contacts the water locking pin roll 323; the water locking pin roll 323 moves downward the very bottom to fully flatten the water-flowing inner tube 322, and the water-flowing inner tube 322 changes from the "OPEN" state to the "Closed" state, thus stopping the water flow.

When suction is required, use a finger to block the negative pressure control port 6 to drain the waste fluid and flushing fluid, and control the suction force of the suction tube 2 by the size of the blocked negative pressure control port 6.

### Embodiment 4

The operation method of the flushing and suction drainage tube described in Embodiment 2 includes not only the operation method described in Embodiment 3 but also the following steps:
When using the flushing and suction drainage tube, remove the insulating strip 24 to make the elastic pin 23 contact the positive and negative poles of the battery 212, thus enabling the battery 212 to energist the light emitting diode to emit light. The light propagates through the optical fiber cable 213 to the head end of the flushing and suction drainage tube for lighting the operative site.

Although the present application has been described in detail with general explanations and specific embodiments, some modifications or improvements can be made on the basis of the present application, which is obvious for those skilled in the art. Therefore, those modifications or improvements without departing from the spirit of the present application are within the scope of protection of the present invention.

## Claims

1. A disposable flushing and suction drainage tube, **characterized in that** it comprises a flushing tube (1), a suction tube (2), and a mounting base (3); one end of the mounting base (3) is connected with the flushing tube (1) and the suction tube (2), while the other end is connected with a gas joint (4) and a water joint (5); the mounting base (3) comprises a tube body base (31) and a control base (32); one end of the tube body base (31) is installed with the flushing tube (1) and the suction tube (2), while the other end is detachably connected with the control base (32);
the control base (32) comprises a control base body (321), a water-flowing inner tube (322), a water locking pin roll (323), and a water control switch (324); the water control switch (324) is slidably mounted onto the control base body (321), the water locking pin roll (323) is movably mounted inside the control base body (321), the water-flowing inner tube (322) is mounted inside the control base body (321), the water locking pin roll (323) and the water control switch (324) cooperate with each other during use to control the water flow in the water-flowing inner tube (322); and the control base body (321) is equipped with a negative pressure control port (6).

2. The disposable flushing and suction drainage tube according to claim 1, **characterized in that** a water-flowing inner cavity (311) and a first gas-flowing inner cavity (312) are arranged inside the tube body base (31), the end of the tube body base (31) distal from the flushing tube (1) is connected with a water route connecting base (7) and a gas route connecting base (8).

3. The disposable flushing and suction drainage tube according to claim 2, **characterized in that** a second gas-flowing inner cavity (80) connected with the first gas-flowing inner cavity (312) is arranged inside the gas route connecting base (8); a connecting guide rail (9) and an annular limiting bulge (10) are arranged outside the gas route connecting base (8); and the gas route connecting base (8) is arranged with a notch (81) corresponding to the position of the negative pressure control port (6).

4. The disposable flushing and suction drainage tube according to claim 3, **characterized in that** a negative pressure channel (11) connected with the second gas-flowing inner cavity (80) is arranged in the control base body (321), a positioning groove (110) corresponding to the connecting guide rail (9) and a groove corresponding to the limiting bulge (10) are arranged inside one end of the negative pressure channel (11), while the gas joint (4) is connected to the other end.

5. The disposable flushing and suction drainage tube according to claim 2, **characterized in that** an inner tube groove (12) is arranged inside the control base body (321), the water-flowing inner tube (322) is installed in the inner tube groove (12); one end of the water-flowing inner tube (322) is installed on the water route connecting base (7), while the other end is connected to the water joint (5); both sides of the inner tube groove (12) are arranged with the longitudinally opened first guide slot (13) and second guide slot (14), one end of the water locking pin roll (323) is installed inside the first guide slot (13), while the other end is installed inside the second guide slot (14).

6. The disposable flushing and suction drainage tube according to claim 1, **characterized in that** the flushing and suction drainage tube further comprises a lighting device (21); the lighting device (21) comprises a light emitting diode (211), a battery (212), and an optical fiber cable (213); the light emitting diode (211), battery (212), and optical fiber cable (213) are connected by electric circuits; the light emitting diode (211) and the battery (212) are installed in the tube body base (31), the front end of the tube body base (31) is arranged with a light hole (22), one end of the optical fiber cable (213) passes through the light hole (22) and then is connected to the light emitting diode (211), while the other end extends in the direction away from the tube body base (31);
the positive and negative pins of the light emitting diode (211) are connected to the positive and negative poles of the battery (212) correspondingly; one pin of the light emitting diode (211) is installed with an elastic pin (23); the elastic pin (23) and the battery (212) are separated by an insulating strip (24); during use, the insulating strip (24) is removed to make the elastic pin (23) contact the positive and negative poles of the battery (212), thus enabling the battery (212) to energise the light emitting diode (211) to emit light;
the position on the tube body base (31) corresponding to the battery (212) is installed with a stop block (25).

7. The disposable flushing and suction drainage tube according to claim 1, **characterized in that** the control base (32) further comprises a fixing shell (325); the fixing shell (325) is detachably mounted onto the control base body (321), the fixing shell (325) and the control base body (321) form a mounting groove, and the water control switch (324) is installed in the mounting groove.

8. The disposable flushing and suction drainage tube according to claim 7, **characterized in that** the water control switch (324) comprises an operating panel (3241), a mounting slider (3242), and a pin roll control block (3243); the operating panel (3241), mounting slider (3242), and pin roll control block (3243) are connected successively from up to down; the pin roll control block (3243) is an inverted rectangular trapezoidal block, which comprises an oblique isosceles surface (15) and a lower bottom surface (16), and the bottom surface of the pin roll control block (3243) has an opening (17).

9. The disposable flushing and suction drainage tube according to claim 1, **characterized in that** the front end of the flushing tube (1) is sloped and separated 5-35 mm from the front end of the suction tube (2); the flushing tube (1) is also connected to a casing pipe (18), and the casing pipe (18) is arranged outside the suction tube (2).

10. A method of operating the flushing and suction drainage tube described in any one of claims 1 to 9, **characterized in that** the operation method includes the steps of:
connect the gas joint (4) to the negative pressure source, and connect the water joint (5) to the water source;
when flushing is required, slide the water control switch (324) to the "ON" end; the water control switch (324) slides to change from the state where its lower bottom surface (16) contacts the water locking pin roll (323) to the state where its oblique isosceles surface (15) contacts the water locking pin roll (323), the water locking pin roll (323) moves upward under the elastic force of the water-flowing inner tube (322), the water-flowing inner tube (322) changes from the "Closed" state to the "OPEN" state, the water flows through the water joint (5) and the water-flowing inner tube (322) and reaches the flushing tube (1) to start the flushing; adjust the position of the water control switch (324), adjust the position where the oblique isosceles surface (15) of the water control switch (324) contacts the water locking pin roll (323), control the upward movement distance of the water locking pin roll (323), and control the flattening degree of the water-flowing inner tube (322), thus realizing the water flow control. When flushing needs to be stopped, slide the water control switch (324) to the "OFF" end; the water control switch (324) slides to change from the state where its oblique isosceles surface (15) contacts the water locking pin roll (323) to the state where its lower bottom surface (16) contacts the water locking pin roll (323); the water locking pin roll (323) moves downward to flatten the water-flowing inner tube (322), and the water-flowing inner tube (322) changes from the "OPEN" state to the "Closed" state, thus stopping the water flow;
when suction is required, use a finger to block the negative pressure control port (6) to drain the waste fluid and flushing fluid, and control the suction force of the suction tube (2) by the size of the blocked negative pressure control port (6).
